# EUROPEAN PATENT SPECIFICATION

(11) **EP 2 744 509 B1**
(45) Date of publication and mention of the grant of the patent: **13.07.2016**
(21) Application number: 12745842.0
(22) Date of filing: 07.08.2012
(51) Int. Cl.: A61K 38/21, A61P 17/14, A61K 8/64, A61Q 7/00

(54) **USE OF INTERFERON BETA FOR THE TREATMENT OF ALOPECIA AREATA**
VERWENDUNG VON INTERFERON-BETA ZUR BEHANDLUNG VON KREISRUNDEM HAARAUSFALL
UTILISATION DE L'INTERFÉRON BÊTA POUR LE TRAITEMENT DE LA PELADE

(30) Priority: 18.08.2011 DE 102011052816
(43) Date of publication of application: 25.06.2014
(73) Proprietor: Tigo GmbH, 65193 Wiesbaden (DE)
(72) Inventor: GRIGOLEIT, Hans-Günther, 65193 Wiesbaden (DE); IVANOV, Ivan G., 1517 Sofia (BG)
(74) Representative: WSL Patentanwälte Partnerschaft mbB
(86) International application number: PCT/EP2012/065451
(87) International publication number: WO 2013/023961

(56) References cited:
- US-A- 5 709 853
- SCHMUTZ J -L: "Expert response: Interferon beta and hair loss", NOUVELLES DERMATOLOGIQUES 2009 LES NOUVELLES DERMATOLOGIQUES FRA, vol. 28, no. 4 PART 1, 2009, page 212, XP9162459, ISSN: 0752-5370
- GALAN-GUTIERREZ M ET AL: "Update on the Treatment of Alopecia Areata", ACTAS DERMOSIFILIOGRAFICAS (ENGLISH EDITION), ELSEVIER, AMSTERDAM, NL, vol. 100, no. 4, 1 May 2009 (2009-05-01), pages 266-276, XP026952383, ISSN: 1578-2190 [retrieved on 2009-05-01]
- SMITH ET AL: "Off-label uses of biologics in dermatology: Interferon and intravenous immunoglobulin (Part 1 of 2)", JOURNAL OF THE AMERICAN ACADEMY OF DERMATOLOGY, C.V. MOSBY, ST. LOUIS, MO, US, vol. 56, no. 1, 24 December 2006 (2006-12-24), pages e1-e54, XP005807803, ISSN: 0190-9622, DOI: 10.1016/J.JAAD.2006.06.016
- FREYSCHMIDT-PAUL P ET AL: "Th-1 cytokines in alopecia areata pathogenesis: IFN-gamma is essentially involved while IL-2 plays a minor role.", ARCHIVES OF DERMATOLOGICAL RESEARCH, vol. 295, no. 8-9, February 2004 (2004-02) , page 345, XP002683101, & 31ST ANNUAL MEETING OF THE ARBEITSGEMEINSCHAFT DERMATOLOGISCHE FORSCHUNG IN COOPERATION WITH THE DEU; BERLIN, GERMANY; FEBRUARY 26-28, 2004 ISSN: 0340-3696
- CHANG DEOK KIM ET AL: "Interferon [beta] Secreted from Human Hair Dermal Papilla Cells Inhibits the Growth of Outer Root Sheath Cells Cultured in Vitro", BIOCHEMICAL AND BIOPHYSICAL RESEARCH COMMUNICATIONS, vol. 290, no. 3, 1 January 2002 (2002-01-01), pages 1133-1138, XP55037181, ISSN: 0006-291X, DOI: 10.1006/bbrc.2001.6324

## Description

The present invention relates to a medicament for the treatment of alopecia areata (AA).

The term "alopecia" means "absence of hair from areas where it is normally present". AA is a non-scarring, inflammatory, hair loss disease that is seen in men and women of all ages, races and in children. This condition is commonly manifested by patchy areas or complete hair loss (A. totalis) on the scalp and other body parts. In severe cases, AA can progress to complete loss of all body hair (A. universalis).

The prevalence (cases at any given point in time) in population is 0.1 - 0.2 %. About 1.7% of the population presents at least one episode of AA during their life. The gender distribution indicates a 1:1 male to female distribution, with a peak incidence (44 %) in the age group 15 to 29 years. 30 % of patients will eventually progress to alopecia totalis (54 % of children and 24 % of adults). The proportion of patients presenting with alopecia totalis declines with each decade of life. The overall incidence of relapse or of experiencing another episode of AA is 86 %.

AA is a disease of the anagen (growth) stage hair follicles. There is substantial evidence that it is an interferon gamma mediated autoimmune disease in which the body attacks its own hair follicles and suppresses or stops hair growth. It has been found that T cell lymphocytes cluster around affected follicles, causing inflammation and subsequent hair loss. Follicles are in a state called dystrophic anagen phase, where follicles are producing root sheath material and often hair fibres - but they are too small and aberrant to protrude above the skin epidermis.

While not life threatening, AA nonetheless has serious implications for the patient and his/her family/social group members because of its psychological and sociological backlash. Although a benign disorder, quality of life, particularly in young patients is seriously impaired, mainly by altering self-perception, self-esteem and social life interference.

There is a chance of recovery from AA without any treatment. However, 34 % of the patients never recover from the initial episode. Up to now there is no causal therapy available for subjects suffering from AA. Limited success has been achieved with the use of corticosteroids, phototherapy in combination with photosensitizing agents, contact immunotherapy and hair transplants.

Galan-Gutierrez et al. present an update on the treatment of alopecia areata" (Actas Dermosifiliograficas (english edition) vol. 100, no. 4, 1 may 2009, pages 266-276).

Freyschmidt-Paul et al. dicuss the role of th-1 cytokines in alopecia areata pathogenesis. Their conclusion is that IFN-gamma is essentially involved while IL-2 plays a minor role (Archives of Dermatological Research, vol. 295, no. 8-9, february 2004, page 345, & 31st Annual meeting of the Arbeitsgemeinschaft Dermatologische Forschung, february 26-28, 2004).

Schmutz discloses in "Expert Response: Interferon beta and Hair Loss" (Nouvelles Dermatologiques, vol. 28, no. 4 part 1, 2009, page 212) that treatment with interferon beta may cause diffuse alopecia.

Smith et al. discuss the off-label uses of biologics in dermatology, particularly with respect to interferon and intravenous immunoglobulin (Journal of the American Academy of Dermatology vol. 56, no. 1, 24 december 2006, pages e1-e54).

Chang et al. postulate that interferon beta secreted from human hair dermal papilla cells inhibits the growth of outer root sheath cells cultured in vitro (Biochemical and Biophysical Research Communications, vol. 290, no. 3, 1 january 2002, pages 1133-1138).

US 5 709 853 A is directed to pharmaceutical compositions for atopic disease comprising interferon beta as an active ingredient.

In light of the above there is a strong need for an efficaceous therapy for the treament of AA, and thus, it is the object of the present invention to provide a means for efficaceaously treating AA in patients suffering from AA.

This object is solved by the present invention by using interferon beta as a medicament in the treatment of AA.

Interferons function as messengers responsible for the communication between cells to induce defence-mechanisms in order to eradicate pathogens or tumor cells. These proteins are produced when pathogens such as bacteria, viruses or tumor cells have been detected in the body by the immune system.

Interferon beta (IFN-β) is a type I interferon produced by fibroblasts and endothelial cells. Interferon beta is mainly involved in the immune response against infections. There are three different interferon beta sources: interferon beta may either be derived from mammalian cells (interferon beta-1a) or from E.coli (interferon beta-1b). Furthermore, there exists human interferon beta.

The present inventors have found that interferon beta is useful as a medicament in the treatment of AA, whereas interferon alpha, which is the other type I interferon is known to be capable to induce AA.

There is substantial scientific evidence that interferon gamma plays a crucial role in the pathogenesis of AA as, e.g. it is described as a biomarker of the disease, or that its presence in human skin grafts promotes hair loss. By using interferon beta as a medicament in the treatment of AA according to the present invention the hair loss associated with AA can be reduced or even stopped. Apparently interferon beta interacts with the high interferon gamma levels in the affected skin area or the body as a whole. Without wishing to be bound by this theory, this interaction may be the reason for the beneficial effect of interferon beta in the treatment of AA.

The type of AA that can be treated by administering the above mentioned interferon beta can be any AA selected from the group consisting of alopecia areata (patchy hair loss), alopecia areata totalis (hair loss of the total scalp) and alopecia areata universalis (total hair loss expanding over the entire body).

The interferon beta of the present invention may be administered to the subject suffering from AA topically or systemically. In cases of only locally occurring AA (patchy hair loss) local administration is preferred, whereas in case of AA universalis systemical administration seems to be preferable. For AA totalis either local or systemical administration is preferred.

In cases where topical administration is realized said administration is preferable achieved by administering the interferon beta formulated in an ointment, a lotion, a gel, a spray, a plaster (sustained or non-sustained release plaster) etc. The formulation can comprise liposomes or nanoparticles as carrier structures for the interferon beta.

For topical administration, the effective interferon beta dose range for treatment of AA is from 10 to 200 µg/day, preferably in the range of from 20 to 150 µg/day. Preferably said dose is split up in 3 to 5 dose units applied separately during the day. The treatment duration depends on the result achieved and may vary in the range of from 1 to 6 weeks. In one particular preferred embodiment of the invention 4.5 - 30µg interferon beta-1b are admininstered topically 5 times daily up to 4 weeks.

In the case of topical administration, dermal penetration is preferably enhanced by applying ultrasound (sonophoresis) to the treated areas.

As regards the inventive embodiments, where interferon beta is administered to the subject to be treated systemically this is done by infusion, subcutaneous or intramuscular injection, preferably.

For systemical administration, the effective interferon beta dose range for treatment of AA is from 0.1 to 125 µg/day, preferably in the range of from 1 to 20 µg/day. In one embodiment of the invention said dose is administered on a daily basis. In other embodiments said dose is not administered on a daily basis but every 2^{nd} day, 3 times a week or only in one dosage unit a week. The treatment duration depends on the result achieved and may vary in the range of from 1 to 6 weeks. In one particular preferred embodiment of the invention 15 - 750 µg human interferon beta are administered up to 6 days per infusion. In another embodiment of the invention 0.25µg interferon beta-1 b is administered subcutaneously every 2^{nd} day. In yet another embodiment of the invention 8.8-22µg interferon beta-1a is administered subcutaneously 3 times a week. In a further embodiment of the invention 30µg interferon beta-1 a is administered intramuscularly once a week.

The interferon beta for use as a medicament in the treatment of AA according to the present invention may be administered in combination with another active agent useful in the treatment of AA. In one embodiment of the invention the administration of interferon beta and the other active agent takes place simultaneously. In another embodiment the administration takes place sequentially or even separately within a certain period of time.

One of the preferred active agents useful in the treatment of AA which can be combined with the interferon beta, is an active agent selected from the group of statins, such as the 3-hydroxy-3-methylglutaryl coenzyme A (HMG-CoA) reductase inhibitors atorvastatin, fluvastatin, pravastatin, lovastatin, simvastatin, pitavastatin and rosuvastatin. In the present invention said statins are preferably applied in daily oral dosages of from 1 to 100 mg/day, more preferably in daily dosages of from 10 to 80 mg/day.

In some embodiments of the invention there is used more than one specific interferon beta in the treatment of AA, namely a combination of two or more interferon betas, such as interferon beta-1 a plus an interferon beta 1 b, or interferon beta-1 a in combination with human interferon beta, or interferon beta-1b in combination with human interferon beta. In one embodiment the two or more interferon betas are administered simultaneously. In other embodiments the administration takes place sequentially or even separately within a certain period of time.

The present invention encompasses compositions comprising interferon beta for use as a medicament in the treatment of AA. Said compositions may comprise other active agents, such as the aforementioned other active agents useful in the treatment of AA. Furthermore, said compositions may comprise in addition or alternatively at least one auxiliary agent selected from the group consisting of carriers, recipients, adjuvants, diluents, and disintegrants.

Particularly, the at least one auxiliary agent may be selected from the group consisting of liposomes, nanoparticles, carboxymethyl cellulose, hydroxyethyl cellulose, mineral oil, petrolatum, glycerine, polysorbat 80, hydroxyethyl starch, dextran, and polyethylene glycol.

Further encompassed by the present invention is the use of interferon beta or the use of a composition comprising interferon beta in the manufacture of a medicament for treatment of AA.

In light of the above the present invention refers also to standard dosage units comprising or being comprised of the aforementioned compositions. Preferred standard dosage units are, in case of topical administration preferably plasters, and in case of systemical administration preparations for infusion or injection (either subcutaneous or intramuscular).

For topical administration, the standard dosage unit comprises preferably 2 to 200 µg interferon beta/dosage unit, more preferably the standard dosage unit comprises 10 to 100 µg interferon beta/dosage unit.

In particularly preferred embodiments of the invention the standard dosage unit comprises 5, 10, 20 or 30 µg interferon beta-1/dosage unit.

For systemical administration, the standard dosage unit comprises preferably 0.1 to 125 µg interferon beta/dosage unit, more preferably the standard dosage unit comprises 1 to 20 µg interferon beta/dosage unit.

In particularly preferred embodiments of the invention the standard dosage unit comprises 0.1, 1, 5, 10, 20, 30, 40 or 50 µg interferon beta-1/dosage unit.

In one particularly preferred embodiment of the invention an infusion preparation comprises 15 - 750 µg human interferon beta/500 ml of said preparation. In another particularly preferred embodiment of the invention an injection preparation for subcutaneous injection comprises 1 µg interferon beta-1 b/ml. In yet another particularly preferred embodiment of the invention an injection preparation for subcutaneous injection comprises 100 µg interferon beta-1a/ml. In a further particularly preferred embodiment of the invention an injection preparation for intramuscular injection comprises 30 µg interferon beta-1a/ml.

### Examples:

In the following examples the interferon beta of the present invention is administered to a subject suffering from locally occurring AA (patchy hair loss) for a specific treatment period locally by intradermal injection at a specific dose.

### Example 1

Administering interferon beta-1 b to a subject suffering from locally occurring AA (patchy hair loss) intradermally at a single weekly dose of 15 µg interferon beta-1 b per 10 cm² of treated area for 4 weeks results in the hair loss associated with AA being significantly reduced. 1 week after termination of the treatment the subject starts suffering from increasing hair loss again.

### Example 2

Extending the treatment period of Example 1 to 6 weeks results in the hair loss associated with AA being almost reduced totally. However, even in this case 1 week after termination of the treatment the subject starts suffering from increasing hair loss again.

### Example 3

Administering interferon beta-1 b to a subject suffering from locally occurring AA (patchy hair loss) intradermally at a single daily dose of 4.5 µg interferon beta-1b per 10 cm² of treated area for 4 weeks results in the hair loss associated with AA being almost reduced totally. 1 week after termination of the treatment the subject starts suffering from increasing hair loss again.

### Example 4

By extending the treatment period of Example 3 to 6 weeks at the end of the treatment period there is almost no significant hair loss identifiable. However, 1 week after termination of the treatment the subject starts suffering from increasing hair loss again.

### Example 5

Administering interferon beta-1 b to a subject suffering from locally occurring AA (patchy hair loss) intradermally at a single daily dose of 15 µg interferon beta-1 b per 10 cm² of treated area for 4 weeks results in the hair loss associated with AA being almost reduced totally. However, 1 week after termination of the treatment the subject starts suffering from increasing hair loss again.

### Example 6

By extending the treatment period of Example 5 to 6 weeks there is no significant hair loss identifiable at the end of the treatment period. However, 1 week after termination of the treatment the subject starts suffering from increasing hair loss again.

### Example 7

Administering interferon beta-1b to a subject suffering from locally occurring AA (patchy hair loss) intradermally at a single daily dose of 30 µg interferon beta-1 b per 10 cm² of treated area for 4 weeks results in the hair loss associated with AA being reduced totally. However, 1 week after termination of the treatment the subject starts suffering from increasing hair loss again.

### Example 8

Extending the treatment period of Example 7 to 6 weeks resulted in a prolongation of the time period after which the subject starts suffering from increasing hair loss after termination of the treatment again to 2 weeks.

For the purpose of original disclosure it is to be noted that any features which may be gathered by a skilled person from the present description and the claims, even if only described in connection with particular further features, may be combined individually as well as in arbitrary combinations with any other of the features or groups of features disclosed herein, unless this is explicitly excluded or technical conditions would render such combinations impossible or senseless. The comprehensive, explicit discussion of any combinations of features which might be thought of is dispensed with just for the sake of brevity and legibility of the description and claims.

## Claims

1. Interferon beta for use in the treatment of alopecia areata.

2. Interferon beta for use according to claim 1, wherein the interferon beta is interferon beta-1a, interferon beta-1b or human interferon beta.

3. Interferon beta for use according to claim 1 or 2, wherein the type of alopecia areata to be treated is locally occuring alopecia areata, alopecia areata totalis or alopecia areata universalis.

4. Interferon beta for use according to one of the claims 1 to 3, wherein the interferon beta is administered to the subject to be treated topically or systemically.

5. Interferon beta for use according to one of the claims 1 to 4, wherein the interferon beta is administered to the subject to be treated topically in the form of an ointment, a lotion, a gel, a spray, a plaster or a sustained release plaster, wherein said administration forms optionally contain liposomes or nanoparticles.

6. Interferon beta for use according to one of the claims 1 to 4, wherein the interferon beta is administered to the subject to be treated systemically by intravenous, subcutaneous or intramuscular injection.

7. Interferon beta for use according to one of the claims 1 to 6, wherein the interferon beta is administered to the subject to be treated simultaneously, sequentially or separately together with at least one other active agent useful in the treatment of alopecia areata.

8. Interferon beta for use according to claim 7, wherein the at least one other active agent useful in the treatment of alopecia areata is a statin.

9. Composition for use in the treatment of alopecia areata said composition comprising interferon beta.

10. Composition for use according to claim 9, comprising at least one auxiliary agent selected from the group consisting of carriers, recipients, adjuvants, diluents, and disintegrants.

11. Composition for use according to claim 10, wherein the at least one auxiliary agent is selected from the group consisting of liposomes, nanoparticles, carboxymethyl cellulose, hydroxyethyl cellulose, mineral oil, petrolatum, glycerin, polysorbat 80, hydroxyethyl starch, dextran, and polyethylene glycol.

12. Composition for use according to one of the claims 9 to 11, comprising at least one other active agent useful in the treatment of alopecia areata.

13. Composition for use according to claim 12, wherein the at least one other active agent useful in the treatment of alopecia areata is a statin.

## Patentansprüche

1. Interferon beta zur Verwendung bei der Behandlung von Alopecia areata.

2. Interferon beta zur Verwendung nach Anspruch 1, wobei das Interferon beta Interferon beta-1a, Interferon beta-1b oder humanes Interferon beta ist.

3. Interferon beta zur Verwendung nach Anspruch 1 oder 2, wobei der Typ der zu behandelnden Alopecia areata lokal auftretende Alopecia areata, Alopecia areata totalis oder Alopecia areata universalis ist.

4. Interferon beta zur Verwendung nach einem der Ansprüche 1 bis 3, wobei das Interferon beta dem zu behandelnden Patienten topisch oder systemisch verabreicht wird.

5. Interferon beta zur Verwendung nach einem der Ansprüche 1 bis 4, wobei das Interferon beta dem zu behandelnden Patienten topisch in Form einer Salbe, einer Lotion, eines Gels, eines Sprays, eines Pflasters oder eines Pflasters mit verzögerter Wirkstofffreisetzung verabreicht wird, wobei diese Verabreichungsformen wahlweise Liposome oder Nanopartikel beinhalten.

6. Interferon beta zur Verwendung nach einem der Ansprüche 1 bis 4, wobei das Interferon beta dem zu behandelnden Patienten systemisch durch intravenöse, subkutane oder intramuskuläre Injektion verabreicht wird.

7. Interferon beta zur Verwendung nach einem der Ansprüche 1 bis 6, wobei das Interferon beta dem zu behandelnden Patienten gleichzeitig, sequenziell oder separat zusammen mit mindestens einem anderen Wirkstoff, welcher bei der Behandlung von Alopecia areata nützlich ist, verabreicht wird.

8. Interferon beta zur Verwendung nach Anspruch 7, wobei der mindestens eine weitere Wirkstoff, welcher bei der Behandlung von Alopecia areata nützlich ist, ein Statin ist.

9. Zusammensetzung zur Verwendung bei der Behandlung von Alopecia areata, wobei die Zusammensetzung Interferon beta aufweist.

10. Zusammensetzung zur Verwendung nach Anspruch 9, welche mindestens ein Hilfsmittel, ausgewählt aus der Gruppe bestehend aus Trägern, Empfängern, Hilfsstoffen, Verdünnungsmitteln und Sprengmitteln aufweist.

11. Zusammensetzung zur Verwendung nach Anspruch 10, wobei das mindestens eine Hilfsmittel ausgewählt ist aus der Gruppe bestehend aus Liposomen, Nanopartikeln, Carboxymethylcellulose, Hydroxyethylcellulose, Mineralöl, Petrolatum, Glycerin, Polysorbat 80, Hydroxyethylstärke, Dextran und Polyethylenglycol.

12. Zusammensetzung zur Verwendung nach einem der Ansprüche 9 bis 11, welche mindestens einen weiteren Wirkstoff, welcher bei der Behandlung von Alopecia areata nützlich ist, aufweist.

13. Zusammensetzung zur Verwendung nach Anspruch 12, wobei der mindestens eine weitere Wirkstoff, welcher zur Behandlung von Alopecia areata nützlich ist, ein Statin ist.

## Revendications

1. Interféron bêta pour utilisation dans le traitement de la pelade.

2. Interféron bêta pour utilisation selon la revendication 1, lequel interféron bêta est l'interféron bêta-1a, l'interféron bêta-1 b ou l'interféron bêta humain.

3. Interféron bêta pour utilisation selon la revendication 1 ou 2, dans lequel le type de pelade à traiter est la pelade locale, la pelade décalvante ou la pelade universelle.

4. Interféron bêta pour utilisation selon l'une quelconque des revendications 1 à 3, lequel interféron bêta est administré au sujet à traiter par voie topique ou de manière systémique.

5. Interféron bêta pour utilisation selon l'une quelconque des revendications 1 à 4, lequel interféron bêta est administré au sujet à traiter par voie topique sous la forme d'un onguent, d'une lotion, d'un gel, d'un spray, d'un emplâtre ou d'un emplâtre à libération prolongée, dans lequel lesdites formes d'administration contiennent facultativement des liposomes ou des nanoparticules.

6. Interféron bêta pour utilisation selon l'une quelconque des revendications 1 à 4, lequel interféron bêta est administré au sujet à traiter de manière systémique par injection intraveineuse, sous-cutanée ou intramusculaire.

7. Interféron bêta pour utilisation selon l'une quelconque des revendications 1 à 6, lequel interféron bêta est administré au sujet à traiter simultanément, successivement ou séparément conjointement avec au moins un autre principe actif utile dans le traitement de la pelade.

8. Interféron bêta pour utilisation selon la revendication 7, dans lequel l'au moins un autre principe actif utile dans le traitement de la pelade est une statine.

9. Composition pour utilisation dans le traitement de la pelade, ladite composition comprenant un interféron bêta.

10. Composition pour utilisation selon la revendication 9, comprenant au moins un agent auxiliaire choisi dans le groupe constitué des vecteurs, des récepteurs, des adjuvants, des diluants et des délitants.

11. Composition pour utilisation selon la revendication 10, dans laquelle l'au moins un agent auxiliaire est choisi dans le groupe constitué des liposomes, des nanoparticules, de la carboxyméthylcellulose, de l'hydroxyéthylcellulose, de l'huile minérale, du pétrolatum, de la glycérine, du polysorbate 80, de l'hydroxyéthylamidon, du dextrane et du polyéthylène glycol.

12. Composition pour utilisation selon l'une quelconque des revendications 9 à 11, comprenant au moins un autre principe actif utile dans le traitement de la pelade.

13. Composition pour utilisation selon la revendication 12, dans laquelle l'au moins un autre principe actif utile dans le traitement de la pelade est une statine.
